# EUROPEAN PATENT APPLICATION

(11) **EP 4 339 279 A2**
(43) Date of publication of application: **20.03.2024**
(21) Application number: 24155279.3
(22) Date of filing: 11.06.2018
(51) Int. Cl.: C12N 1/20

(54) **FROZEN ENZYME PELLETS**

(30) Priority: 15.06.2017 EP 17176193
(62) Divisional of application: 18729978.9
(71) Applicant: DSM IP Assets B.V., 6411 TE Heerlen (NL)
(72) Inventor: PRICE, Claire Emile, 6100 AA ECHT (NL); STREEKSTRA, Hugo, 6100 AA ECHT (NL); JOLINK, Fenna Johanna Catharina, 6100 AA ECHT (NL); VAN HEE, Pim, 6100 AA ECHT (NL); GRABINSKI, Dominik Bohdan, 6100 AA ECHT (NL)
(74) Representative: dsm-firmenich IP

(57) **Abstract**

The present invention relates to a frozen enzyme formulation comprising an enzyme. According to another aspect the present invention relates to a starter culture comprising a frozen enzyme formulation and frozen lactic acid bacteria pellets, wherein the frozen enzyme formulation pellets and the frozen lactic acid bacteria pellets are separate pellets or wherein the enzyme and the lactic acid bacteria are in the same pellets. According to yet another aspect the present invention relates to a method for the production of a frozen enzyme formulation. According to another aspect, the present invention relates to the use of the frozen enzyme formulation.

## Description

### Field of the invention

The present invention relates to a frozen enzyme formulation comprising an enzyme. According to another aspect the present invention relates to a starter culture comprising a frozen enzyme formulation and frozen lactic acid bacteria pellets, wherein the frozen enzyme formulation pellets and the frozen lactic acid bacteria pellets are separate pellets or wherein the enzyme and the lactic acid bacteria are in the same pellets. According to yet another aspect the present invention relates to a method for the production of a frozen enzyme formulation. According to another aspect, the present invention relates to the use of the frozen enzyme formulation.

### Background

Enzymes are more and more used in the production of fermented dairy products, such as cheeses and fermented milk products. It is a challenge to dose the correct amount of enzyme in the production of fermented dairy products since the amount needed for the desired effect can be relatively low. Furthermore, the shelf life and stability of enzyme formulations is important since industrial scale fermented dairy production requires sufficient stocks of enzymes. Finally, there is a problem related to co-formulation of the lactic acid bacteria of a starter culture and enzymes. Lactic acid bacteria are commonly formulated in the form of frozen pellets, whereas enzymes are commonly formulated as liquids. The disadvantage is that fermented dairy producers need to source, store and dose the starter culture and enzymes separately.

### Definitions

The term 'pellets' or granula refers to small solid entities formed by dripping, injecting, spraying, pouring or dispersing material into a cooling medium/cryogenic liquid at a suitably low temperature, having an average diameter between 0.01 and 15 mm.

The term "milk" is intended to encompass milks from mammals and plant sources or mixtures thereof. Preferably, the milk is from a mammal source. Mammals sources of milk include, but are not limited to cow, sheep, goat, buffalo, camel, llama, horse or reindeer. In an embodiment, the milk is from a mammal selected from the group consisting of cow, sheep, goat, buffalo, camel, llama, horse and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. Soy bean milk is preferred. In addition, the term "milk" refers to not only whole milk, but also skim milk or any liquid component derived thereof or reconstituted milk.

As used in this specification, the term "fermented dairy product" or "acidified dairy product" is intended to refer to products which are obtained by the multiplication of lactic acid bacteria in a milk base leading to a milk coagulum. The milk preparation used as raw material for the fermentation may be skimmed or non-skimmed milk, optionally concentrated or in the form of powder. Furthermore, this milk preparation may have been subjected to a thermal processing operation which is at least as efficient as pasteurization. The particular characteristics of the various fermented dairy products depend upon various factors, such as the composition of milk base, the incubation temperature, the lactic acid flora and/or non-lactic acid flora. Thus, fermented dairy products manufactured herein include, for instance, various types of regular yoghurt, low fat yogurt, non-fat yoghurt, kefir, dahi, ymer, buttermilk, butterfat, sour cream and sour whipped cream as well as fresh cheeses such as quark and cottage cheese. Petit Suisse is yet another example of a fermented dairy product.

As used in the present specification, the term "yogurt" refers to products comprising lactic acid bacteria such as *Streptococcus salivarius thermophilus* and *Lactobacillus delbruekii* subsp. *bulgaricus,* but also, optionally, other microorganisms such as *Lactobacillus delbruekii* subsp. *lactis, Bifidobacterium animalis* subsp. *lactis, Lactococcus lactis, Lactobacillus acidophilus* and *Lactobacillus casei,* or any microorganism derived therefrom. The lactic acid strains other than *Streptococcus salivarius thermophilus* and *Lactobacillus delbruekii* subsp. *bulgaricus,* are intended to give the finished product various properties, such as the property of promoting the equilibrium of the gut microbiota.

As used herein, the term "yogurt" encompasses set yogurt, stirred yogurt, drinking yoghurt, heat treated yogurt and yogurt-like products. The term "yogurt" encompasses, but is not limited to, yoghurt as defined according to French and European regulations, e.g. coagulated dairy products obtained by lactic acid fermentation by means of specific thermophilic lactic acid bacteria only (i.e. *Lactobacillus delbruekii* subsp. *bulgaricus* and *Streptococcus salivarius thermophilus)* which are cultured simultaneously and are found to be live in the final product in an amount of at least 10 million CFU (colony-forming unit) / g. Preferably, the yogurt is not heat-treated after fermentation. Yogurts may optionally contain added dairy raw materials (e.g. cream) or other ingredients such as sugar or sweetening agents, one or more flavouring(s), fruit, cereals, or nutritional substances, especially vitamins, minerals and fibers. Such yogurt advantageously meets the specifications for fermented milks and yogurts of the AFNOR NF 04-600 standard and/or the codex StanA-Ila-1975 standard. In order to satisfy the AFNOR NF 04-600 standard, the product must not have been heated after fermentation and the dairy raw materials must represent a minimum of 70% (m/m) of the finished product.

In the present context, the terms "fresh cheese", "unripened cheese", "curd cheese" and "curd-style cheese" are used interchangeably herein to refer to any kind of cheese such as natural cheese, cheese analogues and processed cheese in which the protein/ casein ratio does not exceed that of milk.

The term "starter" or "starter culture" as used herein refers to a culture of one or more food-grade micro-organisms, in particular lactic acid bacteria, which are responsible for the acidification of the milk base. Starter cultures may be fresh (liquid), frozen or freeze-dried. Freeze dried cultures need to be regenerated before use. For the production of a yoghurt, the starter is usually added in an amount from 0.01 to 3% by weight of the total amount of milk base. For the production of cheese, lower dosages can be used such as from 0.006% by weight of the total amount of milk base.

As used herein, the term "lactic acid bacteria" (LAB) or "lactic bacteria" refers to food-grade bacteria producing lactic acid as the major metabolic end-product of carbohydrate fermentation. These bacteria are related by their common metabolic and physiological characteristics and are usually Gram positive, low-GC, acid tolerant, non- sporulating, non-respiring, rod-shaped bacilli or cocci. During the fermentation stage, the consumption of lactose by these bacteria causes the formation of lactic acid, reducing the pH and leading to the formation of a protein coagulum. These bacteria are thus responsible for the acidification of milk and for the texture of the dairy product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus salivarius thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

A "cryoprotectant" is defined herein as a substance used to protect cells or tissues from damage during freezing and thawing. The cryoprotectant may be any additive as long as it protects cells or tissues from damage during freezing and thawing.

As used herein, the term "enzyme" intends to cover any enzyme. Based on its mechanism, the frozen enzyme formulation may comprise an oxidoreductase, a transferase, a hydrolyse, a lyase, an isomerase or a ligase. More preferably, the enzyme in the claimed frozen enzyme formulation is an enzyme which is used in industry, for example in biofuel industry, as biological detergent, in the brewing industry, in the dairy industry, in food processing or in the starch industry. Even more preferably, the frozen enzyme formulation comprises a dairy enzyme such as - but not limited to - a lactase, protease, lipase, catalase, aspartic protease, transglutaminase, laccase, tyrosinase, protein deimidase, oxidase (such as lysyloxidase), peroxidase, peptidase or a protein deamidase. Other useful enzymes are enzymes that are involved in the production of or are capable of producing polysaccharides or enzymes which can modify extracellular polysaccharides. Non-limiting examples of such enzymes are alpha-glucanase, beta-glucanase, glucosidase, cellulase, pectinase, galacturonidase, rhamnosidase, xylosidase, fucosidase, arabinosidase, galactosidase glycosyl transferase, glucan sucrase or fructan sucrase.

As used herein, the term lactase (a beta-galactosidase) refers to an enzyme capable of hydrolyzing lactose in glucose and galactose. The herein used lactase may be a neutral lactase or an acid lactase.

As used herein a protease may be an exoprotease or an endoprotease. A preferred endopeptidase is a proline-specific endoprotease. A preferred exopeptidase may be either an aminopeptidase or a carboxypeptidase. Preferably the exopeptidase is a carboxypeptidase.

As used herein the term lipase may refer to an animal or a microbial lipase. Lipases are used mainly in cheese ripening for development of lipolytic flavors.

As used herein the term catalase refers to an enzyme which catalyses the decomposition of hydrogen peroxide.

The aspartic protease may be of animal origin. Preferably, the aspartic protease is produced by a micro-organism (a microbially produced aspartic protease). The microorganism may for instance be Rhizomucor, for instance Rhizomucor miehei or Rhizomucor pussilus or Cryphonectria, for instance Cryphonectria parasitica. The microorganism may also be selected from the genera of Aspergillus, Trichoderma, Penicillium, Fusarium, Humicola, or Kluyveromyces. These microorganisms may for instance be used as host strain. In a preferred embodiment the microorganism is Aspergillus niger, Aspergillus nidulans, Aspergillus oryzae, Kluyveromyces lactis, or Escherichia coli. In an embodiment of the invention, the aspartic protease is a Rhizomucor miehei aspartic protease. The term "Rhizomucor miehei aspartic protease" encompasses the aspartic protease homologously produced in Rhizomucor miehei. A process for the preparation of the enzyme via fermentation is described in US-A-3,988,207. The term "Rhizomucor miehei aspartic protease" also encompasses a recombinant Rhizomucor miehei aspartic protease, for example a Rhizomucor miehei aspartic protease produced in a host organism (e.g. other than Rhizomucor miehei) transformed with DNA coding for the Rhizomucor miehei aspartic protease. A method for the production of a recombinant Rhizomucor miehei aspartic protease in a host organism is described in EP-A-700253. In another embodiment of the invention the aspartic protease is chymosin. Chymosin may for instance be extracted from the stomach of a calf, camel or seal. In a preferred embodiment of the invention the chymosin is produced by a microorganism, e.g. via recombinant DNA technology in bacteria, e.g. Escherichia coli, yeast, e.g. Kluyveromyces lactis, or filamentous fungi, e.g. in Aspergillus niger.

As used herein a transglutaminase refers to an enzyme that is used in food industry for obtaining protein-crosslinks.

As used herein a protein deamidase refers to an enzyme which is capable of converting the amide groups found in the side chain of the amino acids asparagine or glutamine.

As used herein a laccase refers to an enzyme which uses oxygen to act on (poly)phenols, which may lead to the formation of cross-links.

As used herein a tyrosinase refers to an enzyme which uses oxygen to act on tyrosine residues (either free or bound in peptide chains), which may lead to the formation of cross-links.

As used herein a deimidase refers to an enzyme which is capable of converting imide groups such as found in the side-chain of arginine.

As used herein an oxidase refers to an enzyme that oxidizes reduced substrates, using oxygen as an electron acceptor.

As used herein a peroxidase refers to an enzyme that oxides reduced substrates, using a peroxide, usually hydrogen peroxide, as an electron acceptor.

### Detailed description

The present invention relates to a frozen enzyme formulation comprising an enzyme, wherein the frozen enzyme formulation is in the form of frozen pellets. The present inventors found that enzyme formulations frozen in the form of small pellets retain their enzyme activity. It is believed that the quick freezing and thawing of small aliquots of an enzyme formulation is not detrimental to the enzyme activity. This allows the storage of enzyme formulations in frozen form. Surprisingly, the enzyme activity remains high, even in absence of preservatives such as sorbates, benzoates and parabens. In one aspect, the frozen enzyme formulation is free from or low in preservatives such as sorbates, benzoates and parabens. In another aspect, the frozen enzyme formulation is free from the mentioned preservatives. A frozen form is particularly advantageous in industries where the frozen enzyme is combined with microorganisms in frozen form for ease of logistics, storing and dosing.

In a preferred embodiment, the frozen enzyme formulation is a frozen liquid enzyme formulation. A frozen liquid enzyme formulation means that a liquid enzyme formulation is frozen, i.e. is in a solid form. Preferably, the liquid is water.

In a preferred embodiment, the invention provides a frozen enzyme formulation comprising an enzyme, wherein the frozen enzyme formulation is in the form of frozen pellets and wherein the frozen pellets are free flowing pellets, i.e. they are formed by dripping a droplet of liquid enzyme formulation in a cooling gas/cryogenic liquid (the terms are used interchangeably herein) and their shape is not determined by a container which was used during freezing. Moreover, such free flowing pellets can change position with other pellets upon moving/shaking a container comprising a multitude of frozen enzyme pellets. For the avoidance of doubt, frozen precipitates which precipitates are the result of a centrifugation step are not within the scope of the claim, because they are not free flowing and because their shape is determined by the used centrifugation tube. Also for the avoidance of doubt, the frozen enzyme pellets of the invention are not freeze dried.

In yet another embodiment, the invention provides a frozen enzyme formulation comprising an enzyme, wherein the frozen enzyme formulation is in the form of frozen pellets and wherein the water content is at least 20% (w/w). More preferably, the water content is at least 30%, 40%, 50%, 60%, 70%, 80% or at least 90% (w/w). The skilled person is very well capable of determining the water content of a composition.

More preferably, the frozen enzyme formulation is selected from a fermentation broth, a concentrated fermentation broth, unconcentrated liquid phase obtained from a fermentation broth, a micro filtrate from a fermentation broth, an ultra-filtrate from a fermentation broth or a chromatographically purified enzyme solution. Enzymes are typically produced by fermentation of microorganisms which produce the enzyme. The enzyme can be present in the fermentation broth (in case of an extracellular produced enzyme), which can be frozen as such. Alternatively, a fermentation broth is further processed by for example micro filtration or ultrafiltration. The resulting filtrates comprising the enzyme can advantageously be frozen in the form of pellets. In one aspect, the enzyme is purified by using chromatography, i.e. the enzyme in the frozen enzyme formulation is a purified enzyme. The purification is performed to enrich the enzyme of interest and to reduce the other compounds present in a non-chromatographically purified enzyme preparation. Processes for purifying enzyme preparations using chromatography are known per se. Selecting the most appropriate chromatographic separation methods depend on molecular characteristics of both the enzyme of interest and of the relevant side activities present. Relevant molecular characteristics are the isoelectric point, hydrophobicity, molecular surface charge distribution, molecular weight of the relevant enzyme and the side activity as well as several other protein chemical properties. A practical background on the use of these characteristics in selecting the appropriate chromatographic separation process, can be found in for example the Protein Purification Handbook (issued by Amersham Pharmacia Biotech, nowadays GE Healthcare Bio-Sciences, Diegem, Belgium). Suitable chromatographic separation methods comprise ion exchange chromatography, affinity chromatography, size exclusion chromatography, hydrophobic interaction chromatography and others. In case the enzyme is produced intracellularly, the microorganisms are typically (but not necessarily) separated from the liquid phase and the microorganisms are lysed to obtain the produced enzyme and the micro-organisms are typically separated from the liquid phase (comprising the enzyme), i.e. the frozen enzyme formulation can also be a liquid fraction obtained after lysis of the microorganism (which may or which may not have been separated from the fermentation broth) used as the enzyme production host. Preferably, the remainders of the microorganism used as host are removed from the enzyme solution obtained after lyzing the microorganism.

In a preferred embodiment, the pellets have an average diameter of between 0.01 and 15 mm. A pellet of this diameter allows a rapid freezing and thawing of the enzyme which retains the enzyme activity. Preferably, the pellets have an average diameter of between 0.01 and 10 mm or 0.1 and 10 mm. More preferably the present pellets have an average diameter of between 0.1 and 5mm.

Preferably, and as already discussed above, the present pellets are individual pellets, or are individually frozen pellets. Individual pellets are free flowing pellets. The present pellets are preferably made in a liquid cooling gas, more preferably in liquid nitrogen, carbon dioxide or helium. The present pellets are preferably made by dripping a droplet of liquid enzyme formulation in the liquid cooling gas. Therefore, the present pellets have preferably a sphere form. Alternatively, the pellets have a more elongated shape.

Preferably, the frozen enzyme pellets are stored at a temperature below around -40 to -55 degrees Celsius (preferably around -45 to -55 degrees Celsius) until use although lower temperatures can also be used, e.g. around -40 to -80 degrees Celsius.

In one aspect, the frozen enzyme formulation comprises a preservative. A preservative is preferably selected from a salt such as sodium chloride or glycerol. Depending on the particular enzyme and its end use, benzoate can also be used as a preservative. Preferably, the amount of preservative is 0.1 to 80% (w/w) of the frozen enzyme formulation. More preferably, the amount of preservative is 0.1 to 50% (w/w) of the frozen enzyme formulation. Most preferably, the amount of preservative is 1 to 10% (w/w) of the frozen enzyme formulation.

In a preferred embodiment, the enzyme used in a frozen enzyme formulation is produced by a microorganism and said microorganism is present in an amount of less than 5×10⁴ colony forming units per gram frozen enzyme formulation. The microorganism might be present in the frozen enzyme formulation in case the microorganisms was used for the production of the enzyme and could not be completely separated from the enzyme. An example of a microorganism is a microorganism from the species *Aspergillus, Bacillus or Kluyveromyces* or any other suitable industrial host strain. Preferably, the amount of microorganisms per gram frozen enzyme formulation is less than 1×10⁴ cfu, more preferably less than 1×10³ cfu, even more preferably less than 1×10³ cfu, most preferably less than 1×10² cfu, most preferably the amount of microorganisms is zero cfu. Preferably, the present frozen enzyme formulation does not comprise lactic acid bacteria. To avoid any misunderstanding, the enzyme in the frozen enzyme formulation is not contained in a production host cell, but is separated therefrom via methods known in the art. I.e. in case the enzyme of interest is intracellular produced in a microorganism, the host cell processed such that the enzyme is released from the interior of the production microorganism and processed such that the remainders of the microorganism are removed from the enzyme of interest.

In yet another aspect, the formulation comprises less than 5×10⁴ colony forming units of any microorganism per gram frozen enzyme formulation, i.e. the formulation is low in or free from contaminating microorganisms. Preferably, the amount of contaminating microorganisms per gram frozen enzyme formulation is less than 1×10⁴ cfu, more preferably less than 1×10³ cfu, even more preferably less than 1×10³ cfu, most preferably less than 1×10² cfu, most preferably the amount of contaminating microorganisms is zero cfu.

In a preferred embodiment, the present enzyme is a lactase, protease, lipase, catalase, aspartic protease, transglutaminase, laccase, tyrosinase, protein deimidase, oxidase (such as lysyloxidase), peroxidase, peptidase, protein deamidase, glycosyl transferase, glucan sucrase or fructan sucrase. Preferred enzymes are lactase and/or chymosin.

In yet another aspect, the invention provides a frozen enzyme formulation comprising an enzyme, wherein the frozen enzyme formulation is in the form of at least 10, preferably at least 100, more preferably at least 1000 and most preferred at least 10000 frozen pellets. Preferably, the frozen pellets are contained in a storage container suitable for storage between -40 and -80 degrees Celsius, more preferably between -40 and -55 degrees Celsius and even more preferably between -45 and -55 degrees Celsius.

Given the advantageous combination of the present frozen enzyme formulation with frozen lactic acid bacteria, the present invention also relates to a starter culture comprising a frozen enzyme formulation as defined herein and frozen lactic acid bacteria pellets, wherein the frozen enzyme formulation pellets and the frozen lactic acid bacteria pellets are separate pellets. The combination of enzyme pellets and bacteria pellets provides an improved use since the pellets can be sourced, stored and used together. The present inventors found that there is no negative influence of the enzyme pellets to the bacteria pellets and vice versa. An example of a potential negative influence would be the attack on proteins on the surface of the starter culture bacteria by a protease if the starter culture and the protease would be formulated together in the same pellet. Similarly, other enzymes could have other specific targets at the bacterial surface. Depending on the enzyme, it is advantageous to have a formulation in which the frozen enzyme pellets and the frozen lactic acid bacteria pellets are separate pellets. The frozen pellets do not interact and remain individual pellets during storage. The present starter culture allows the producer of fermented dairy products to inoculate milk with both lactic acid bacteria and the correct amount of enzyme in one go. In one aspect, the frozen enzyme pellets and the frozen lactic acid bacteria are part of a kit comprising separate containers for the frozen enzyme formulation and for the frozen lactic acid bacteria. This allows the combination of different amounts of enzyme and lactic acid bacteria pellets.

In yet another aspect, the invention provides a starter culture comprising a frozen enzyme formulation, wherein the frozen enzyme formulation and the frozen lactic acid bacteria are present in the same pellet. Such frozen pellets can for example be prepared by mixing lactic acid bacteria and an enzyme composition and subsequently producing frozen pellets therefrom. Such an embodiment is especially useful in cases wherein the enzyme and the lactic acid bacteria do not have a negative impact on each other.

Preferably, the starter culture comprises at least 50 gram of frozen material, such as at least 100 gram frozen material, such as at least 250 gram frozen material, such as at least 500 gram frozen material, most preferably at least 900 gram frozen material. Preferably the starter culture comprises less than 500 kg frozen material. The advantage of using at least 50 gram material is that the amount of lactic acid bacteria in the industry of fermented dairy products is sufficient.

In a preferred embodiment, the present frozen lactic acid bacteria pellets have a content of viable cells of at least 1×10⁷ colony forming units per gram frozen lactic acid bacteria pellets, more preferably at least 1×10⁸ cfu/g, more preferably at least 1×10⁹ cfu/g, even more preferably at least 1×10¹⁰ cfu/g, yet even more preferably at least 1×10¹¹ cfu/g, in particular at least 1×10¹² cfu/g and more in particular at least 1×10¹³ cfu/g. The advantage of concentrated or highly concentrated lactic acid bacteria pellets is that small amounts of pellets is sufficient for the inoculation of large amounts of milk.

In a preferred embodiment, the present frozen lactic acid bacteria pellets comprise a cryoprotectant. Examples of cryoprotectants include, but are not limited to, sugars (*e.g.* sucrose, fructose, trehalose), polyalcohols (*e.g.* glycerol, sorbitol, mannitol), polysaccharides (*e.g.* celluloses, starch, gums, maltodextrin), polyethers (*e.g.* polypropylene glycol, polyethylene glycol, polybutylene glycol), antioxidants (*e.g.* natural antioxidants such as ascorbic acid, beta-carotene, vitamin E, glutathione, chemical antioxidants), oils (*e.g.* rapeseed oil, sunflower oil, olive oil), surfactants (*e.g.* Tween^{®}20, Tween^{®}80, fatty acids), peptones (*e.g.* soy peptones, wheat peptone, whey peptone), tryptones, vitamins, minerals (*e.g.* iron, manganese, zinc), hydrolysates (*e.g.* protein hydrolysates such as whey powder, malt extract, soy), amino acids, peptides, proteins, nucleic acids, nucleotides, nucleobases (*e.g.* cytosine, guanine, adenine, thymine, uracil, xanthine, hypoxanthine, inosine), yeast extracts (*e.g.* yeast extracts of *Saccharomyces spp., Kluyvermomycesa spp.,* or *Torula spp*.), beef extract, growth factors, and lipids.

In one aspect, the ratio of the number of frozen enzyme formulation pellets and the number of frozen lactic acid bacteria pellets in the present starter culture can vary on the desired use, for example such ratio is around 1: 1 or equal or less than 0.5 or equal or more than 2. As mentioned above, the invention also provides a kit comprising at least 2 containers in which the different pellets are present and allowing the user to make any desired combination of the amount of lactic acid pellets and enzyme pellets.

In a preferred embodiment, the lactic acid bacteria is selected from the group consisting of *Lactobacillus* spp., *Bifidobacterium* spp., *Streptococcus* spp., *Lactococcus* spp. *Leuconostoc* spp., *Pediococcus* spp. and *Propionobacterium* spp.. More preferably, the present lactic acid bacteria is selected from the group consisting of *Lactobacillus delbruekii* subsp. *bulgaricus, Streptococcus salivarius thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.* Most preferably the present lactic acid bacteria are *Lactobacillus delbruekii* subsp. *bulgaricus* and *Streptococcus salivarius thermophilus.*

According to another aspect, the present invention relates to a method for producing a frozen enzyme formulation in the form of frozen pellets, comprising the steps of providing a liquid enzyme formulation and freezing the liquid enzyme formulation in the form of frozen pellets. Preferably, the step of freezing the liquid enzyme formulation in the form of frozen pellets comprises freezing droplets of the liquid enzyme formulation in a cooling gas/cryogenic liquid. Preferably, the cooling gas/cryogenic liquid is liquid nitrogen, carbon dioxide or helium. The present liquid enzyme formulation can be a fermentation broth, or a micro or ultra-filtrate thereof. More preferably, the present liquid enzyme formulation is directly contacted with the cooling gas/cryogenic liquid without a container present between the liquid enzyme formulation and the cooling gas/cryogenic liquid. Preferably, said method is performed at large scale, i.e. at least 100 kg/hour, more preferably at least 150 kg/hour. The obtained frozen pellets are preferably stored at a temperature between -40 and -80 degrees Celsius, preferably between -40 and -55 degrees Celsius, more preferably at a temperature between -45 and -55 degrees Celsius.

According to another aspect, the present invention relates to the use of the frozen enzyme formulation as defined herein in a starter culture, preferably in a starter culture for the production of a fermented dairy product.

More preferably, the present invention relates to the use of the frozen enzyme formulation as defined herein for the production of a fermented dairy product.

The invention further provides a method for producing a food product, preferably a dairy product, comprising adding a frozen enzyme pellet to a substrate used for the production of a food product and subsequently performing all other necessary steps for preparing said food product.

The invention is further illustrated with the following non-limiting examples.

### EXAMPLE 1

### Frozen MAXILACT^{®} LGi

A small aliquot (~15 ml) of ccUF (i.e. a concentrated sample obtained for example via ultrafiltration) samples with an activity of between 10800 NLU/g and 14000 NLU/g were frozen and determined on activity after thawing. The residual activity of both samples was 100%. Increasing the volume frozen, decreased the activity after thawing. ccuF is an enzyme formulation after cell lysis, separation, clarification and ultrafiltration. It contains no preservatives. A typical ccUF comprises around 5 to 10% (w/w) of enzyme.

| **Sample #** | **Description/ Quantity** | | **Yield** |
|---|---|---|---|
| 1 | | ccUF 'as is' | 100% |
| 2 | | 50 ml | 81% |
| 3 | | 500 ml | 54% |
| 4 | | 500 ml | 54% |
| 5 | | 500 ml | 58% |
| 6 | | 500 ml | 60% |
| 7 | | 5 liter | 57% |

### EXAMPLE 2

### Frozen MAXILACT^{®} LGi pellets

MAXILACT^{®} ccUF* droplets were dropped in liquid nitrogen using an Eppendorf pipet (1000 µl) with drops of 10 to 15 µl. This resulted in droplets with a diameter varying from -250 µm to 3 mm. This material was subsequently defrosted and activity determined. The yield on freeze was 89%.

### EXAMPLE 3

### Frozen MAXIREN^{®} 600 pellets

MAXIREN^{®} 600 droplets were dropped in liquid nitrogen using a syringe. 10-15 ml of liquid enzyme formulation was added at a time to the syringe and the liquid dropped into the liquid nitrogen by gravity only, no pressure was applied. This resulted in droplets with a diameter ~ 3 mm. The material was stored at -45°C for 10 months. Samples were removed at regular intervals, defrosted and activity determined. The residual activity was stable over 10 months and comparable to the original liquid form of MAXIREN^{®} 600.

| **Storage time of frozen pellets** | | **Activity Maxiren^{®}600 liquid (IMCU/ml)** | **Activity MAXIREN^{®} 600 frozen pellets (IMCU/ml)** |
|---|---|---|---|
| | 1 day | 549 | 545 |
| | 4 weeks | 540 | 530 |
| | 4 months | 533 | 527 |
| | 6,5 months | 533 | 560 |
| | 10 months | 537 | 579 |

## Claims

1. Frozen enzyme formulation comprising an enzyme, wherein the frozen enzyme formulation is in the form of frozen pellets.

2. Frozen enzyme formulation according to claim 1, wherein the frozen enzyme formulation is a frozen liquid enzyme formulation.

3. Frozen enzyme formulation according to claim 1 or 2, wherein said frozen pellets are free flowing pellets.

4. Frozen enzyme formulation according to any of the preceding claims, wherein the water content is at least 20% (w/w).

5. Frozen enzyme formulation according to any of the preceding claims, wherein the frozen enzyme formulation is selected from a fermentation broth, a concentrated fermentation broth, unconcentrated liquid phase obtained from a fermentation broth, a micro filtrate from a fermentation broth, an ultra-filtrate from a fermentation broth or a chromatographically purified enzyme solution.

6. Frozen enzyme formulation according to any of the preceding claims, wherein the pellets have an average diameter of between 0.01 and 15 mm.

7. Frozen enzyme formulation according to any of the preceding claims, further comprising a preservative.

8. Frozen enzyme formulation according to any of the preceding claims, wherein said enzyme is produced by a microorganism and wherein said microorganism is present in an amount of less than 5×10⁴ colony forming units per gram frozen enzyme formulation.

9. Frozen enzyme formulation according to any of the preceding claims, wherein said formulation comprises less than 5×10⁴ colony forming units of any microorganism per gram frozen enzyme formulation.

10. Frozen enzyme formulation according to any of the preceding claims, wherein the enzyme is selected from the group consisting of lactase, protease, lipase, catalase, aspartic protease, transglutaminase, laccase, tyrosinase, protein deimidase, oxidase, peroxidase, peptidase or a protein deamidase.

11. Starter culture comprising a frozen enzyme formulation according to any of the preceding claims and frozen lactic acid bacteria pellets, wherein the frozen enzyme formulation pellets and the frozen lactic acid bacteria pellets are separate pellets.

12. Starter culture according to claim 11, wherein the frozen lactic acid bacteria pellets have a content of viable cells of at least 1×10⁷ colony forming units per gram frozen lactic acid bacteria pellets.

13. Starter culture according to claim 11 or 12, wherein the frozen lactic acid bacteria pellets further comprise a cryoprotectant.

14. Starter culture comprising a frozen enzyme formulation according to any of the preceding claims and frozen lactic acid bacteria, wherein the frozen enzyme formulation and the frozen lactic acid bacteria are present in the same pellets.

15. Starter culture according to claim 14, wherein the pellets have a content of viable cells of at least 1×10⁷ colony forming units per gram pellets.

16. Starter culture according to claim 14 or 15, wherein the pellets further comprise a cryoprotectant.

17. Starter culture according to any of the claims 11 to 16, wherein the lactic acid bacteria is selected from the group consisting of *Lactobacillus* spp., *Bifidobacterium* spp., *Streptococcus* spp., *Lactococcus spp..Leuconostoc* spp, *Pediococcus* spp. and *Propionobacterium* spp..

18. Method for the production of frozen enzyme formulation in the form of frozen pellets, comprising the steps of providing a liquid enzyme formulation and freezing the liquid enzyme formulation in the form of frozen pellets.

19. Method according to claim 18, wherein the freezing comprises freezing droplets of said liquid enzyme formulation in a cooling gas/cryogenic liquid.

20. Method according to claim 18 or 19, further comprising storing the obtained frozen pellets at a temperature between -40 and -80 degrees Celsius, preferably between -40 and -55 degrees Celsius, more preferably at a temperature between -45 and -55 degrees Celsius.

21. Use of the frozen enzyme formulation according to any of the claims 1 to 10 in a starter culture, preferably in a starter culture for the production of a fermented dairy product.

22. Use of the frozen enzyme formulation according to any of the claims 1 to 10 for the production of a fermented dairy product.
